# EUROPEAN PATENT APPLICATION

(11) **EP 2 096 146 A1**
(43) Date of publication of application: **02.09.2009**
(21) Application number: 09002523.0
(22) Date of filing: 23.02.2009
(51) Int. Cl.: C09B 61/00, A23L 1/275, A23L 2/58

(54) **Deodorized plant pigment derived from Ipomoea Batatas**

(30) Priority: 29.02.2008 JP 2008049108; 29.02.2008 JP 2008049109; 29.02.2008 JP 2008049119
(71) Applicant: SAN-EI GEN F.F.I., INC., Toyonaka, Osaka 561-8588 (JP)
(72) Inventor: Nishiyama, Koji, Osaka 5618588 (JP); Ichi, Takahito, Osaka 5618588 (JP); Onishi, Hironori, Osaka 5618588 (JP); Hamasaki, Koji, Osaka 5618588 (JP); Uchida, Koji, Osaka 5618588 (JP)
(74) Representative: Müller-Boré & Partner Patentanwälte

(57) **Abstract**

The present invention provides a colorant derived from *Ipomoea Batatas* that is completely free of an odor originating from *Ipomoea Batatas,* or in which the odor has been significantly reduced, and a colorant formulation containing the colorant. The present invention also provides a method for producing the odorless or low-odor colorant derived from *Ipomoea Batatas*.

The colorant of the invention derived from *Ipomoea Batatas* can be prepared by subjecting an adsorption-treated product of an *Ipomoea Batatas* colorant extract to at least one treatment selected from adsorption, ion exchange, acid treatment, enzyme treatment, and membrane separation, so that the concentration of the aroma components contained therein is 150 ppm or less when the color value is E(10%/1 cm) = 160.

## Description

### TECHNICAL FIELD

The present invention relates to a plant colorant that is derived from *Ipomoea Batatas* (purple sweet potato) of the genus *Ipomoea* within the family *Convolvulaceae,* which is odorless or whose odor has been significantly reduced to only a faint odor; and to a colorant formulation containing the colorant. More particularly, the invention relates to a plant colorant derived from *Ipomoea Batatas* that is odorless or has only a faint odor, in which the development of an odor over time due to long-term storage or the effects of light or heat has been significantly reduced, and in which precipitation over time has been significantly reduced; and to a colorant formulation containing the colorant. Moreover, the invention relates to a method for producing the odorless or low-odor plant colorant.

Furthermore, the invention relates to foods colored with the plant colorant, and to a coloring method therefor.

### BACKGROUND ART

Various synthetic coloring agents such as Food Red No. 2 (Amaranth) , Food Red No. 3 (Erythosine) , Food Red No. 40 (Allura Red) , Food Red No. 102 (New Coccine) , Food Red No. 104(Phloxine), Food Red No. 105(Rose Bengale), Food Red No. 106(Acid Red), carmoisine (azorubine), Citrus Red No. 2 New Red, etc., have heretofore been used as coloring agents for various foods because of their excellent light resistance and ability to impart bright colors to foods. With the recently growing trend toward the use of natural products, however, the use of these synthetic coloring agents is becoming less frequent.

Examples of known natural colorants used to impart red to purplish red colors to foods include anthocyanin colorants such as red cabbage color, grape juice color, grape skin color, purple corn color, and berry color ; quinone colorants such as cochineal extract and lac color, gardenia red beet red color, and monascus color. However, at a pH of 5 or less, quinone colorants such as cochineal color change color from yellow to orange, and cannot be colored red to purplish red. Monascus color and beet red color are both poor in light and heat resistance, and undergo noticeable discoloration. Gardenia red is a purplish, dark red color; therefore, it is difficult to impart a bright red to purplish red color using this colorant. Grape juice color, grape skin color, purple corn color, and berry color are not also usable to impart a bright red to purplish red color. They are also significantly poor in light resistance.

Conversely, plants of the genus *Ipomoea* within the family *Convolvulaceae* (hereinafter also referred to as the "plants of the genus C. *Ipomoea"*), which are anthocyanin colorants, and more specifically, a colorant derived from *Ipomoea Batatas* (hereinafter also referred to as *"Ipomoea Batatas* colorant"), can impart a bright red to purplish red color, and are also excellent in light and heat resistance. Therefore, these colorants are widely used mainly in coloring foods such as beverages.

Known methods for preparing the *Ipomoea Batatas colorants* are as follows: a method wherein tuberous roots (ground, chipped, or the like) of *Ipomoea Batatas* are extracted using an acidic solvent (water and/or an alcohol) (Japanese Unexamined Patent Publication Nos. 62-297363, 62-297364, and 07-227246); a method wherein an enzyme such as cellulase, amylase, or pectinase is added during the extraction using the acidic solvent (water and/or an alcohol) (Japanese Unexamined Patent Publication No. 04-103669); and a method wherein an anthocyanin colorant obtained from a colorant solution of *Ipomoea Batatas* is purified by contacting the colorant solution of *Ipomoea Batatas* with an anion exchange resin (Japanese Unexamined Patent Publication No. 04-154871). WO 04/78741 discloses a method for obtaining a colorant component (cyanidin) contained in *Ipomoea Batatas,* in which an acidic solvent extract of tuberous roots (ground, chipped, or the like) of *Ipomoea Batatas* undergoes adsorption, ion exchange, membrane separation, pH adjustment, extraction, or salt precipitation.

However, known *Ipomoea, Batatas* colorants have a characteristic vegetable odor or aroma that originates from the *Ipomoea Batatas* used as the starting material. When these colorants are used in products such as, e.g., foods or cosmetics, the odor or aroma may adversely affect the flavor, taste, or smell of these products. Moreover, known *Ipomoea Batatas* colorants have been indicated as undergoing a so-called "odor regression" phenomenon: an aroma develops after heating or long-term storage, and gradually becomes stronger (Japanese Unexamined Patent Publication No. 04-154871).

However, the problem of "odor regression" has yet to be resolved even with various methods.

### DISCLOSURE OF THE INVENTION

### PROBLEM TO BE SOLVED BY THE INVENTION

It is an object of the present invention to provide an *Ipomoea Batatas* colorant that has no adverse effect, over an extended period, on the taste and flavor of various products such as foods, drugs, quasi-drugs, and cosmetics, and that can be added safely to these products.

More specifically, it is a first object of the invention to provide an *Ipomoea Batatas* colorant that is free of an odor originating from *Ipomoea Batatas,* or in which the odor has been significantly reduced, and to provide a colorant formulation containing the colorant. It is a second object of the invention to provide an *Ipomoea Batatas* colorant with excellent stability that undergoes no changes over time, such as so-called "odor regression" or precipitation (clouding or sedimentation), after heating or long-term storage, and to provide a colorant formulation containing the colorant. It is a third object of the invention to provide a method for producing the odorless or low-odor *Ipomoea Batatas* colorant. It is a fourth object of the invention to provide foods and beverages colored with the *Ipomoea Batatas* colorant, and to provide a method for preparing such foods and beverages.

### MEANS FOR SOLVING THE PROBLEM

The inventors conducted extensive research to solve the prior art problems concerning *Ipomoea Batatas* colorants. Consequently, they found that the aroma components contained in *Ipomoea Batatas* that are responsible for the unpleasant or off-odor can be significantly reduced by subjecting an adsorption-treated product of a colorant extract prepared from *Ipomoea Batatas* to at least one treatment selected from the group consisting of adsorption, ion exchange, acid treatment, extraction, enzyme treatment, and membrane separation, thereby yielding a desired odorless or low-odor colorant (an *Ipomoea Batatas* colorant). They also found that the thus-obtained *Ipomoea Batatas* colorant does not undergo "odor regression" or precipitation (clouding or sedimentation) after heating or long-term storage.

On the basis of these findings, the inventors ascertained that by using the *Ipomoea Batatas* colorant prepared according to the above-described method, it is possible to prepare an odorless or low-odor colorant formulation containing an *Ipomoea Batatas* colorant with excellent stability over time, which enables foods and beverages to be stably colored a desired red to purplish red color, without impairing qualities such as flavor, transparency, and the like. The term "purplish red" as used herein means a red color tinged with purple (red is stronger).

The present invention has been accomplished based on the above-described findings.

The invention provides a plant colorant derived from *Ipomoea Batatas* as recited in the following Items (1) and (2).
(1) A plant colorant derived from *Ipomoea Batatas,* wherein a concentration of aroma components contained therein is 150 ppm or less when color value E (10%/1 cm) is 160.
(2) The colorant derived from *Ipomoea Batatas* according to Item (1), wherein the concentration of aroma components is a total concentration of the following components that can be contained in the colorant: 2-methylbutanol, isoamyl alcohol, tridecane, ethyl lactate, hexanol, trans-linalool oxide, benzyl alcohol, phenethyl alcohol, 5-(methylthio)-pentanenitrile, phenylpropanenitrile, ethyl-2-hydroxy-3-phenylpropane, dimethyl phthalate, dihydroactinidiolide, 4-vinylphenol, triethyl citrate, dibutyl phthalate, 4-vinyl-2,6-dimethoxyphenol, phenylacetic acid, ethyl vanillate, 4-ethylcatechol, limonene, cis-pinane, α-terpineol, N-nitrosodibutylamine, guaiacol, 3,7-dimethyloct-1-ene-3,7-diol, 3-hydroxy-α-pyrone, phenol, γ-nonalactone, 4-vinylguaiacol, vanillin, dibutyl phthalate, and acetic acid.
   The invention also provides a colorant formulation containing the colorant derived from *Ipomoea Batatas* of Item (1) or (2). Embodiments of the colorant formulation include those recited in the following Items (3) to (5).
(3) A colorant formulation containing the colorant derived from *Ipomoea Batatas* of Item (1) or (2).
(4) The colorant formulation according to Item (3), which is in the form of a solution.
(5) The colorant formulation according to Item (3) or (4), wherein a content of the colorant derived from *Ipomoea Batatas* is from 1 to 90 wt%.
   The invention relates to foods and beverages as recited in the following Items (6) and (7).
(6) A food or a beverage comprising the colorant formulation of any one of Items (3) to (5), the food or beverage being colored with the colorant formulation.
(7) The food or beverage according to Item (6), which is a beverage or a candy.
   The invention relates to a method for coloring a food or a beverage as recited in the following Items (8) and (9).
(8) A method for coloring a food or a beverage, comprising adding the colorant formulation of any one of Items (3) to (5) to the food or beverage as an ingredient.
(9) The method according to Item (8), wherein the food or beverage is a beverage or a candy.
   Further, the invention relates to a method for producing the above-mentioned highly purified colorant derived from *Ipomoea Batatas* as recited in the following Items (10) to (23).
(10) A method for producing an odorless or low-odor colorant derived from *Ipomoea Batatas,* comprising subjecting an adsorption-treated product of an *Ipomoea Batatas* colorant extract to at least one treatment selected from the group consisting of adsorption, ion exchange, acid treatment, extraction, enzyme treatment, and membrane separation.
(11) The method for producing a plant colorant according to Item (10), wherein the colorant extract of *Ipomoea Batatas* is obtained by extracting while an *Ipomoea Batatas* plant is finely sliced in an acid extraction solvent, or by extracting by immersing a finely sliced *Ipomoea Batatas* plant in an acid extraction solvent.
(12) The method according to Item (11), wherein the acid extraction solvent has a pH of 1 to 4.
(13) The method according to any one of Items (10) to (12), wherein the acid treatment uses an acid that is used as a food additive.
(14) The method according to any one of items (10) to (12), wherein the acid treatment uses at least one inorganic acid selected from the group consisting of sulfuric acid, hydrochloric acid, phosphoric acid, and nitric acid.
(15) The method according to any one of Items (10) to (14), wherein the acid treatment comprises exposing to a pH of 1 to 4 the adsorption-treated product of the *Ipomoea Batatas* colorant extract, or a treated product of the adsorption-treated product after ion exchange, acid treatment, extraction, enzyme treatment, or membrane separation.
(16) The method according to Item (10), wherein the membrane separation is at least one treatment selected from the group consisting of membrane filtering, ultrafiltration, reverse osmosis, electrodialysis, ion selective membrane treatment, and ion exchange.
(17) The method according to Item (10), wherein the enzyme treatment is an acid protease treatment.
(18) A method for producing an odorless or low-odor colorant derived from *Ipomoea Batatas,* comprising removing a high molecular weight compound from an adsorption-treated product of an *Ipomoea Batatas* colorant extract, followed by removal of a low molecular weight compound from the colorant extract by a membrane separation treatment.
(19) The method according to Item (18), wherein the removal of a high molecular weight compound is at least one treatment selected from the group consisting of enzyme treatment, membrane separation, and gel filtration.
(20) The method according to Item (19), wherein the membrane separation treatment used to remove a high molecular weight compound is ultrafiltration using a membrane having a molecular weight cut-off of 10⁴ to 10⁶.
(21) The method according to any one of Items (18) to (20), wherein the acid treatment is performed prior to, subsequent to, or at the same time as the removal of a high molecular weight compound.
(22) The method according to Item (18), wherein the membrane separation treatment is reverse osmosis using a membrane having a molecular weight cut-off of 2,000 to 4,000.

### EFFECTS OF THE INVENTION

In accordance with the present invention, there is provided an *Ipomoea Batatas* colorant with excellent stability that is free of an odor originating from *Ipomoea Batatas* or in which the odor has been significantly reduced, and that undergoes significantly reduced changes over time, such as so-called "odor regression" or precipitation after heating or long-term storage; and a colorant formulation containing the colorant. The colorant and the colorant formulation of the invention can impart a desired red to purplish red color to foods and beverages, drugs, quasi-drugs, cosmetics, and other various products without impairing qualities such as taste, flavor, and transparency, thus providing foods and beverages, drugs, quasi-drugs, and cosmetics that are colored stably and satisfactorily over a prolonged period of time.

### BRIEF DESCRIPTION OF THE DRAWINGS

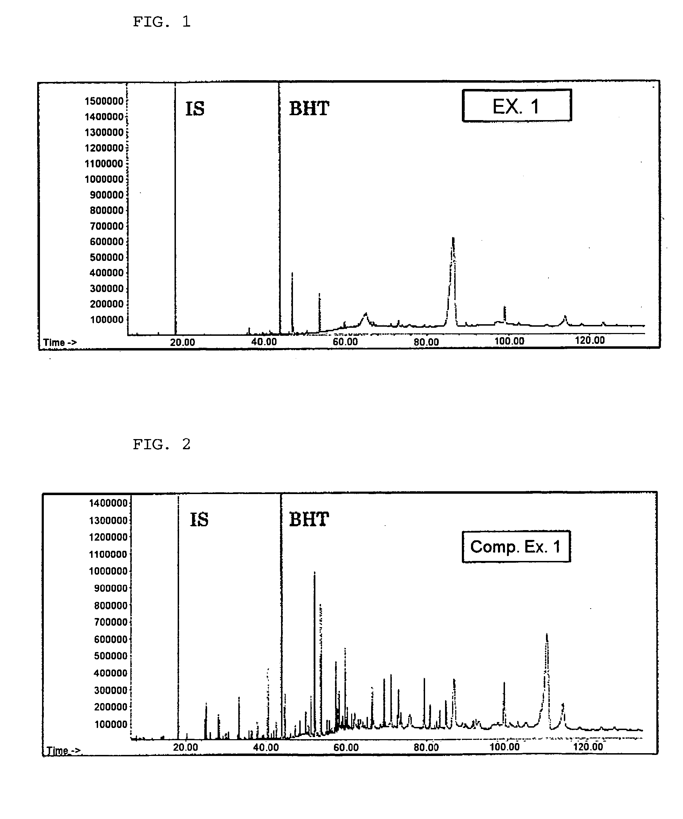

FIG. 1 is a graph showing the results of measuring the amount of aroma components contained in the colorant formulation that contains the *Ipomoea Batatas* colorant that is prepared in Example 1, using a gas chromatography-mass spectrometer (GC-MS); wherein the IS peak represents the peak for 3-heptanol, which is used as an internal standard substance, and the BHT peak represents 2,6-di-t-butyl-4-methyl phenol, which is contained as a stabilizer for diethyl ether; the ordinate represents the abundance, and the abscissa represents the retention time.
FIG. 2 is a graph showing the results of measuring the amount of aroma components contained in the colorant formulation that contains the *Ipomoea Batatas* colorant prepared in Comparative Example 1, using a gas chromatography-mass spectrometer (GC-MS).

### MODES FOR CARRYING OUT THE INVENTION

### (I) Ipomoea Batatas and Preparation Method Thereof

The colorant of the invention is an odorless or low-odor plant colorant derived from *Ipomoea Batatas* that is free of an odor that originates from the *Ipomoea Batatas,* or in which the odor has been significantly reduced.

As used herein, the term "colorant derived from *Ipomoea Batatas*" or *"Ipomoea Batatas* colorant" broadly means colorants prepared from *Ipomoea Batatas* as the starting material.

The color value of "E(10%/1 cm)" as used herein is a numerical value determined as follows: the absorbance is measured for a solution obtained by dissolving the *Ipomoea Batatas* colorant of the invention in McIlvaine buffer (disodium phosphate solution-citric acid solution, pH 3) at the maximum absorption wavelength (near 530 nm) in the visible region (measurement cell width: 1 cm), and the measured absorbance is converted to the absorbance of a 10 w/v% solution containing the *Ipomoea Batatas* colorant.

The term "aroma components" as used herein means various aroma components contained in *Ipomoea Batatas,* especially those responsible for an unpleasant or off-odor. Specific examples include 2-methylbutanol, isoamyl alcohol, tridecane, ethyl lactate, hexanol, trans-linalool oxide, benzyl alcohol, phenethyl alcohol, 5-(methylthio)-pentanenitrile, phenylpropanenitrile, ethyl-2-hydroxy-3-phenylpropane, dimethyl phthalate, dihydroactinidiolide, 4-vinylphenol, triethyl citrate, dibutyl phthalate, 4-vinyl-2,6-dimethoxyphenol, phenylacetic acid, ethyl vanillate, 4-ethylcatechol, limonene, cis-pinane, α-terpineol, N-nitrosodibutylamine, guaiacol, 3,7-dimethyloct-1-ene-3,7-diol, 3-hydroxy-α-pyrone, phenol, γ-nonalactone, 4-vinylguaiacol, vanillin, dibutyl phthalate, and acetic acid.

A feature of the *Ipomoea Batatas* colorant of the invention is that the total concentration of the above-mentioned aroma components that can be contained therein is 150 ppm or less, preferably 100 ppm or less, and more preferably 80 ppm or less, when the color value of the colorant is adjusted to E(10%/1 cm) = 160. The aroma component concentration is substantially proportional to the color value (E (10%/1 cm)).

The *Ipomoea Batatas* colorant of the invention may be completely free of the aroma components (total concentration: 0 ppm), or may contain from one to all of the aroma components, as long as the total concentration falls within the above-mentioned range (150 ppm or less). Moreover, the *Ipomoea Batatas* colorant of the invention is not limited to colorants having the color value (E(10%/1 cm) = 160), and may be any colorant having a total concentration of detected aroma components within the above-defined range when the color value of the colorant is adjusted to the above-defined value.

The *Ipomoea Batatas* colorant of the invention, which has been completely deodorized or deodorized to have only a faint odor by minimizing the content of the aroma components, is obtainable by subjecting an adsorption-treated product of a colorant extract prepared from *Ipomoea Batatas* to any one of adsorption, ion exchange, acid treatment, enzyme treatment, extraction, and membrane separation, or a combination of two or more of any of these treatments.

Accordingly, the present invention provides a method for producing the odorless or low-odor *Ipomoea Batatas* colorant (hereinafter also referred to as *"Ipomoea Batatas* colorant"). From another point of view, the method of the invention can be interpreted as a purification method for an *Ipomoea Batatas* colorant, which is useful in removing or reducing the components contained in the *Ipomoea Batatas* colorant extract that are responsible for an unplesant or off-odor; and more specifically, removing or reducing the following components: 2-methylbutanol, isoamyl alcohol, tridecane, ethyl lactate, hexanol, trans-linalool oxide, benzyl alcohol, phenethyl alcohol, 5-(methylthio)-pentanenitrile, phenylpropanenitrile, ethyl-2-hydroxy-3-phenylpropane, dimethyl phthalate, dihydroactinidiolide, 4-vinylphenol, triethyl citrate, dibutyl phthalate, 4-vinyl-2,6-dimethoxyphenol, phenylacetic acid, ethyl vanillate, 4-ethylcatechol, limonene, cis-pinane, α-terpineol, N-nitrosodibutylamine, guaiacol, 3,7-dimethyloct-1-ene-3,7-diol, 3-hydroxy-α-pyrone, phenol, γ-nonalactone, 4-vinylguaiacol, vanillin, dibutyl phthalate, and acetic acid. From yet another point of view, the method of the invention can also be interpreted as a deodorization method for an *Ipomoea Batatas* colorant.

The colorant extract of *Ipomoea Batatas* for use in the method of the invention can be prepared by solvent extraction of a portion of an *Ipomoea Batatas* plant containing a targeted colorant, and more specifically, an anthocyanin colorant.

Examples of usable portions of the plant include roots, stems, leaves, fruit (seeds), petals, buds, and the like that contain an anthocyanin colorant. The callus of *Ipomoea Batatas* is also included within the above-mentioned plant. The plant portion may undergo an extraction operation as is (raw) or as a ground product (coarsely powdered, finely chopped, or a like form), or may be ground (e.g., powdered), as required, after being dried, and then undergo an extraction operation.

Preferable examples of solvents used in the extraction include, but are not limited to, alcohols, water, and mixtures thereof. Examples of alcohols include C₁-C₄ lower alcohols such as methanol, ethanol, propanol, isopropyl alcohol, butanol, and the like. Preferably, the solvent is water or a hydrous alcohol. The hydrous alcohol preferably has an alcohol content of 40 vol% or less.

Usable as a solvent for extraction is an acidic solution, and more specifically, an acidic solution adjusted to a pH of 1 to 4, and preferably 1 to 3. The acidic solution can be prepared by adding to the extraction solvent, an inorganic acid such as hydrochloric acid, sulfuric acid, nitric acid, or phosphoric acid, or an organic acid such as citric acid, acetic acid, malic acid, or lactic acid. The amount of the inorganic or organic acid in the extraction solvent is not limited as long as the pH of the solvent is within the above-defined range; however, adjusting the amount suitably within the range of 0.01 to 10 wt% is preferable.

The extraction method may be any generally used method. Examples of usable extraction methods include, but are not limited to, cold or hot immersion of an *Ipomoea Batatas* plant or a portion thereof (in an unprocessed form, or a coarsely powdered or chopped form), or a dried product thereof (including a dried and sliced product, e.g., a powder or a like ground product); extraction by heating with stirring, followed by filtration to obtain an extract; and percolation.

Extraction is preferably performed under acidic conditions. Extraction under acidic conditions can be performed by subjecting an *Ipomoea Batatas* plant or a portion thereof (in an unprocessed form, or a coarsely powdered or chopped form), or a dried product thereof (including a dried and sliced product, e.g., a powder or a like ground product) to any of the above-mentioned extraction treatments using an acid extraction solvent. Preferable examples of such extraction methods include extraction while a colorant-containing portion of *Ipomoea Batatas* (raw or dried) is finely sliced in an acid extraction solvent; and a method wherein a colorant-containing portion of *Ipomoea Batatas* (raw or dried) is finely sliced, and the finely sliced product is subsequently immersed in an acid extraction solvent to perform an extraction. In this case, the extraction treatment may be performed using an acid extraction solvent at 100°C or less. One example of this extraction method is a method that includes immersing a finely sliced product of a colorant-containing portion of *Ipomoea Batatas* (raw or dried) in an acid extraction solvent having a pH of 1 to 4, followed by treatment at 100°C or less, and preferably at room temperature (about 10 to 30°C).

The resulting extract is prepared into a colorant extract by optionally removing the solids therefrom via solid-liquid separation such as filtration, coprecipitation, or centrifugal separation. This colorant extract is subsequently subjected to adsorption treatment either directly or after being concentrated. The adsorption treatment can be performed according to a routine method, for example, using activated carbon, silica gel, porous ceramics or the like; styrene-based Duolite S-861, Duolite S-862, Duolite S-863 or Duolite S-866 (trademarks of the U.S.A. Diamond Shamrock Corp.); aromatic Sepabeads SP70, Sepabeads SP700 or Sepabeads SP825 (trademarks of Mitsubishi Chemical Co.); Diaion HP10, Diaion HP20, Diaion HP21, Diaion HP40 or Diaion HP 50 (trademarks of Mitsubishi Chemical Co.), or a synthetic adsorption resin such as Amberlite XAD-4, Amberlite XAD-7 or Amberlite XAD-2000 (trademarks of Organo Co. Ltd.).

The adsorption-treated product for use in the invention can be recovered by washing the adsorption carrier, such as a resin on which the colorant component in the colorant extract had been adsorbed during the adsorption'treatment, in a suitable solvent, e. g., a hydrous alcohol. Water containing about 1 to about 20 vol% ethanol is usually preferable as a hydrous alcohol.

The resulting adsorption-treated product of the *Ipomoea Batatas* colorant extract is subsequently subjected to various purification treatments such as adsorption, ion exchange, acid treatment, enzyme treatment, extraction, and membrane separation. These purification treatments may be performed alone or in combination. Preferably used are at least one of treatment selected from the group consisting of adsorption, ion exchange, acid treatment, enzyme treatment, and membrane separation; and more preferably used are at least one of treatment selected from the group consisting of acid treatment, enzyme treatment, and membrane separation are used.

Examples of adsorption treatments are as mentioned above.

The ion exchange treatment is not limited, and may be performed according to a routine method using a conventional ion exchange resin (a cation or anion exchange resin). Non-limiting examples of cation exchange resins include Diaion SK1B, Diaion SK102, Diaion SK116, Diaion PK208, Diaion WK10, and Diaion WK20 (trademarks of Mitsubishi Chemical Co.); and non-limiting examples of anion exchange resins include Diaion SA10A, Diaion SA12A, Diaion SA20A, Diaion PA306, Diaion WA10, and Diaion WA20 (trademarks of Mitsubishi Chemical Co.).

The acid treatment of the invention can be accomplished by exposing the adsorption-treated product of the *Ipomoea Batatas* colorant extract, or a treated product obtained by further subjecting the adsorption-treated product to any of the various treatments (such as adsorption, ion exchange, extraction, enzyme treatment, and membrane separation), to acidic conditions having a pH of 1 to 4, and preferably a pH of 1 to 3. Preferably, the acid treatment is performed, for example, after the removal of high molecular weight components from the adsorption-treated product by enzyme treatment or membrane separation, or is performed at the same time as these treatments.

Specifically, the acid treatment can be performed simply by adding an acid to the treated product, and adjusting the pH of the product to the above-defined range. The acid may be any acid selected from those generally used as food additives. Examples of such acids include organic acids such as citric acid, acetic acid, malic acid, and lactic acid; and inorganic acids such as sulfuric acid, hydrochloric acid, phosphoric acid, and nitric acid. Preferably, the acid treatment uses an inorganic acid that is generally used as a food additive. The temperature of the acid treatment is not limited, and a suitable temperature can be usually selected from the range of 5 to 100°C. For example, the temperature ranges from 20 to 100°C or from 40 to 100°C. Preferably, the temperature ranges from 40 to 80°C. The acid treatment time is also not limited, and a suitable time can be usually selected from the range of 1 to 300 minutes. In general, the higher the temperature of the acid treatment is, the shorter the treatment time can be; for example, in the case of an acid treatment at 40 to 100°C, the treatment time can be selected from the range of 5 to 60 minutes. The treated product may be optionally stirred during the acid treatment.

The enzyme treatment of the invention is performed in order to remove by decomposition the high molecular weight components contained in the adsorption-treated product of the *Ipomoea Batatas* colorant extract, or in a treated product obtained by further subjecting the adsorption-treated product to any of the various treatments (such as adsorption, ion exchange, extraction, enzyme treatment, and membrane separation).

Preferably, the enzyme treatment can be performed by allowing an acid protease to act on the adsorption-treated product of the *Ipomoea Batatas* colorant extract or the acid-treated product thereof. When an acid protease is acted on a product that is the adsorption-treated product or the treated product except acid-treated product (i.e., a product after adsorption, ion exchange, extraction, or membrane separation, etc.), the above-mentioned acid is preferably added to the treated product beforehand in order to adjust the pH thereof under acidic conditions having a pH of about 1 to 6, and preferably about 3 to about 6.

An acid protease of any origin may be used herein as long as it acts under acidic conditions. Specific examples of commercially available acid proteases include acid proteases of origins such as *Aspergillus niger, Aspergillus saitoi, Rhizopus niveus, Rhizopus delemar, Penicillium duponti,* and the like (see the homepage of the National Food Research Organization (Japan)).

The treatment temperature and time can be suitably selected according to the type of protease used. The treatment temperature is typically from 35 to 80°C, preferably from 40 to 60°C, and more preferably from 40 to 50°C. The treatment time is not limited, and a suitable time can usually be adjusted within the range of 5 minutes to 24 hours.

In the invention, the extraction treatment is not limited, and may be performed by a method that includes contacting the adsorption-treated product of the *Ipomoea Batatas* colorant extract, or a treated product obtained by subjecting the that adsorption-treated product to any of the various treatments (such as adsorption, ion exchange, acid treatment, enzyme treatment, and membrane separation), with carbon dioxide, ethylene, propane or the like in liquid form, within a sealed apparatus at a temperature and pressure equal to or higher than the critical point.

The term "membrane separation treatment" as used herein broadly means filtration methods using membranes. Examples of such treatments include filtration using functional polymer membranes such as a membrane filter (MF) membrane, an ultrafiltration (UF) membrane, a reverse osmosis membrane, and an electrodialysis membrane. Known membrane separation treatments include, in addition to the ultrafiltration method and reverse osmosis method using the above membranes, a dialysis method utilizing a concentration gradient across an ion separation membrane, and an electrodyalysis method wherein an ion exchange membrane is used as a separation memberane and a voltage is applied to the membrane. For industrial purposes, the reverse osmosis membrane separation method is preferred. The membrane for use in these membrane separation methods may be made of any of natural, synthetic and semi-synthetic materials. Examples of such materials include cellulose, cellulose di- or tri-acetate, polyamide, polysulfone, polystyrene, polyimide, and polyacrylonitrile.

The membrane separation treatment used in the invention includes a method for separating and removing high molecular weight compounds using a membrane having a molecular weight cut-off of, for example, 10⁴ to 10⁶; and a method for separating and removing low molecular weight compounds using a membrane having a molecular weight cut-off of about 2,000 to about 4,000, preferably about 3,000. Specific examples of the former method include a method using an ultrafiltration (UF) membrane such as NTU-3150 membrane, NTU-3250 membrane, NTU-3550 membrane or NTU-3800 UF membrane (produced by Nitto Denko Corp.); Cefilt-UF (produced by Nippon Gaishi Inc.); or AHP-2013 membrane, AHP-3013 membrane or AHP-1010 membrane (produced by Asahi Kasei Corp.). Specific examples of the latter method include a method using a reverse osmosis membrane (having a molecular weight cut-off of about 3,000) such as NTR-7250 membrane, NTR-7410 membrane, NTR-7430 membrane or NTR-7450 membrane (produced by Nitto Denko Corp.); or AIP-3013 membrane, ACP-3013 membrane, ACP-2013 membrane, AIP-2013 membrane or AIO-1010 membrane (produced by Asahi Kasei Corp.).

These treatments may be performed alone or in combination, or the same treatment may be repeated under the same or different conditions.

A preferred membrane separation treatment is, but is not limited to, a method that includes removing high molecular weight components such as proteins from the adsorption-treated product of the *Ipomoea Batatas* colorant extract (removal of high molecular weight compounds), followed by removing low molecular weight compounds from the treated product by separation. This method can also include an acid treatment. The acid treatment may be performed prior to, subsequent to, or at the same time as the removal of high molecular weight compounds. When performed at the same time as the removal of high molecular weight compounds, the acid treatment may be, for example, an acid protease treatment, as described later in Example 3.

The removal of high molecular weight compounds can be performed effectively by the above-described enzyme treatment or the membrane separation treatment utilizing an ultrafiltration membrane or the like. In this case, a membrane separation treatment using a membrane having a molecular weight cut-off of about 10⁴ to about 10⁶, which is for use in removing high molecular weight compounds by separation, can be suitably employed. Note, however, that the removal of high molecular weight compounds can also be performed according to a routine method such as gel filtration, in place of the above methods.

Examples of preferred treatment methods include a method wherein the treated product, from which high molecular weight compounds have been removed, is subsequently treated with an acid, and the thus-obtained treated product is subjected to a membrane separation treatment to remove low molecular weight compounds therefrom by separation; and a method wherein a treated product that has simultaneously undergone an acid treatment and a removal of high molecular weight compounds is subjected to a membrane separation treatment to remove low molecular weight compounds therefrom by separation.

The removal of high molecular weight compounds herein may be performed by, for example, an enzyme treatment, preferably an acid protease treatment, or by ultrafiltration using a membrane having a molecular weight cut-off of about 10⁴ to about 10⁶. The separation and removal of low molecular weight compounds may be performed by, for example, reverse osmosis using a membrane preferably having a molecular weight cut-off of 2,000 to 4,000, and more preferably about 3,000.

In the thus-obtained *Ipomoea Batatas* colorant of the invention, the above-mentioned aroma components originating from *Ipomoea Batatas* that can cause an unpleasant or off-odor have been removed effectively. Accordingly, there is provided an odorless or low-odor plant colorant whose odor has been reduced to the extent that the addition of the colorant to a food will have little effect on the flavor thereof. Moreover, the *Ipomoea Batatas* colorant of the invention obtained by the above-described method undergoes no changes over time, such as "odor regression" after heating or long-term storage. This is believed to be a result of the decomposition or removal of the precursors of the aroma components that can emit an unpleasant or off-odor by the method of the invention.

### (II) Colorant Formulation

The *Ipomoea Batatas* colorant of the invention can be prepared as a colorant formulation in the form of a solution containing the colorant dissolved or dispersed (emulsified) in water, an alcohol such as ethanol or propylene glycol, or another solvent; or as a dry colorant formulation (such as a powder, granules, tablets, pills, or the like). A solution form is preferred. The invention thus provides a colorant formulation containing, as an active ingredient, the above-described *Ipomoea Batatas* colorant.

The colorant formulation may solely contain the *Ipomoea Batatas* colorant of the invention, or may contain, in addition to the *Ipomoea Batatas* colorant, carriers and various additives acceptable from the standpoint of food hygiene. Specific examples of such carriers and additives include dextrin, lactose, and powdered syrup, as well as preservatives (such as sodium acetate and protamine), stabilizers (such as sodium phosphate and sodium metaphosphate), antioxidants (such as rutin and ascorbic acid), and other food additives generally used in colorants and colorant formulations.

When the colorant formulation of the invention contains various carriers, additives, and the like, the amount of the *Ipomoea Batatas* colorant in the colorant formulation may be, for example, typically from 1 to 90 wt%, and preferably from 10 to 60 wt%. The colorant formulation of the invention is useful as a red- or purplish red-based coloring agent for foods and beverages, drugs, quasi-drugs, cosmetics, feed, etc., and especially as a natural coloring agent.

### (III) Method for Coloring Foods and Beverages

In the thus-obtained plant colorant of the invention, the aroma components originating from *Ipomoea Batatas,* which can cause an unpleasant or off-odor, have been removed effectively. Therefore, when used as a coloring agent for a food or a beverage, the colorant of the invention does not affect the original flavor thereof. Moreover, the colorant has excellent light and heat resistance, and can stably impart a bright red to purplish red color to the food or beverage. Furthermore, the above-described plant colorant of the invention exhibits significantly reduced precipitation (clouding or sedimentation); therefore, when used for coloring a food or a beverage, it does not impair the appearance or qualities thereof over time. Hence, the above-described plant colorant of the invention and the colorant formulation are particularly suitable for use in coloring foods and beverages.

The invention relates to a method for coloring foods and beverages using the *Ipomoea Batatas* colorant or the colorant formulation containing the colorant. The method of the invention for coloring foods and beverages using the plant colorant or the colorant formulation is not limited, and can be accomplished by adding the plant colorant or the colorant formulation during the manufacturing process.

The *Ipomoea Batatas* colorant or the colorant formulation used in coloring may be in any form. For example, the colorant or colorant formulation may be in the form of a liquid; or in the form of a powder obtained by adding an excipient such as dextrin, lactose, or the like into the liquid, and spray-drying the mixture; or in the form of an emulsion obtained by emulsification or double-emulsification of the liquid using an emulsifier such as, e.g., a glycerine fatty acid ester, a sorbitan fatty acid ester, or gum arabic.

The *Ipomoea Batatas* colorant of the invention and the colorant formulation can be widely used for coloring general foods and beverages. Examples of such foods and beverages include beverages; confectionaries such as candies, jellies, frozen deserts, chewing gums, and the like; strawberry and other jams and fillings; pickles and seasonings; etc., with beverages and candies being preferred.

Beverages targeted by the invention include soft drinks such as sports drinks; vegetable juices and fruit beverages containing fruit, vegetable, and like juices; carbonated beverages such as cola, ginger ale, and cider; coffee and tea beverages such as black tea and green tea; milk beverages such as cocoa and lactic acid bacteria beverages; alcoholic beverages such as liqueurs, cocktails, fruit liqueurs, and *chu-hai* (cocktails based on liquors made from sweet potatoe or rice); etc. Examples of preferred beverages are acidic beverages with a pH of 1.5 to 6.8, and particularly 2 to 4.

Beverages are typically prepared by mixing major ingredients such as saccharides, fruit juices, acids, and the like with stabilizers, flavoring agents, and the like. Brightly colored red to purplish red beverages can be prepared by adding to these beverages the plant colorant of the invention or the colorant formulation, followed by mixing, pasteurization, and cooling, and subsequently filling containers with the mixtures.

In the manufacturing process of such beverages, a heat sterilization process suitable for the type of beverage is employed, such as hot-pack filling typically at 93°C; plate sterilization of 2 seconds at 130°C; UHT pasteurization; or retort sterilization at an F value of 25. The *Ipomoea Batatas* colorant or the colorant formulation used in the invention has heat resistance comparable to that of synthetic coloring agents, and is also very stable under the foregoing pasteurization conditions. This makes the *Ipomoea Batatas* colorant or the colorant formulation suitable for use in coloring beverages.

The amount of the *Ipomoea Batatas* colorant of the invention or the colorant formulation used in the beverage is not limited, and may be any amount that can impart a desired color to the beverage. For example, the amount of the *Ipomoea Batatas* colorant (E(10%/1 cm) = 160) per 100 wt% of the beverage may be from 0.0005 to 1.0 wt%, and preferably from 0.001 to 0.5 wt%.

Examples of candies targeted by the invention include drops, butterscotch, and other hard candies; caramels, nougats, marshmallows, and other soft candies; starch candies; gummy candies; etc.

The use of the *Ipomoea Batatas* colorant of the invention or the colorant formulation enables these candies to be colored bright red to purplish red. Depending on the type, some candies may require high transparency along with bright coloring. The use of the plant colorant of the invention or the colorant formulation, which exhibits significantly reduced precipitation (clouding or sedimentation), enables the preparation of candy that satisfies the above requirement. Furthermore, the use of the *Ipomoea Batatas* colorant or the colorant formulation, in which the odor characteristic of the colorant has been significantly reduced, enables the preparation of candy that has an original flavor and exhibit an excellent flavor release.

Of these candies, hard candy can be prepared as follows. Any glucide such as a sugar, a starch syrup, any of various sugar alcohols, or the like is blended into water and dissolved by heating to about 150°C, after which the solution is cooled to about 130°C. During the addition of a flavoring agent and various acidulants, the colorant of the invention is added to the cooled solution and mixed, a mold such as a container is filled with the resulting mixture to be molded, and the mixture is solidified by cooling. Soft candy can be prepared as follows. A mixture of ingredients such as a saccharide, a polysaccharide thickener, and other optional ingredients is dissolved in water, and the solution is heated to boiling and subsequently cooled to about 100°C. A usual emulsifier such as a fat or oil, or an emulsifier is added to the solution and mixed, and then a flavoring agent and the colorant of the invention are added to the mixture. The mixture is then subsequently crystallized, or the boiled solution is filled into a mold. Gummy candy can be prepared as follows. A saccharide such as a starch syrup, sugar, or the like, along with optional ingredients such as a polysaccharide thickener, are boiled, and the colorant of the invention is added when sub-ingredients such as gelatin, fruit juice, an acidulant, a flavoring agent, and the like are added as required. The resulting mixture is then filled into a mold.

During the manufacturing process, these candies are exposed to an extremely high temperature because the temperature at which the fluidity needed for filling and molding is attained is generally as high as about 100 to about 160°C. The colorant of the plant of the genus C. *Ipomoea* or the colorant formulation used in the invention has heat resistance comparable to that of synthetic coloring agents, and is very stable at high temperatures as mentioned above. This makes the *Ipomoea Batatas* colorant or the colorant formulation suitable for use in coloring candy.

The amount of the *Ipomoea Batatas* colorant or the colorant formulation used in the candy is not limited, and may be any amount that can impart a desired color to the candy. For example, the amount of the *Ipomoea Batatas* colorant (E(10%/1 cm) = 160) per 100 wt% of the candy may be from 0.0005 to 1.0 wt%, and preferably from 0.001 to 0.5 wt%.

The invention includes the following embodiments.
(a) A method for purifying a colorant derived from *Ipomoea Batatas,* comprising subjecting an adsorption-treated product of an *Ipomoea Batatas* colorant extract to at least one treatment selected from the group consisting of adsorption, ion exchange, acid treatment, enzyme treatment, extraction, and membrane separation.
(b) A method for purifying a colorant derived from *Ipomoea Batatas,* comprising removing a high molecular weight compound from an adsorption-treated product of an *Ipomoea Batatas* colorant extract, followed by a membrane separation treatment to remove a low molecular weight compound from the treated product.
(c) The method according to Item (b), wherein the membrane separation treatment is reverse osmosis using a membrane having a molecular weight cut-off of 2,000 to 4,000.
(d) The purification method according to Item (b), wherein the removal of a high molecular weight compound is at least one treatment selected from the group consisting of enzyme treatment, membrane separation, and gel filtration.
(e) A method for deodorizing a colorant derived from *Ipomoea Batatas,* comprising subjecting an adsorption-treated product of an *Ipomoea Batatas* colorant extract to at least one treatment selected from the group consisting of adsorption, ion exchange, acid treatment, enzyme treatment, extraction, and membrane separation.
(f) A method for deodorizing a colorant derived from *Ipomoea Batatas,* comprising removing a high molecular weight compound from an adsorption-treated product of an *Ipomoea Batatas* colorant extract, followed by a membrane separation treatment to remove a low molecular weight compound from the treated product.
(g) The deodorization method according to Item (f), wherein the membrane separation treatment is reverse osmosis using a membrane having a molecular weight cut-off of 2,000 to 4,000.
(h) The deodorization method according to Item (f), wherein the removal of a high molecular weight compound is at least one treatment selected from the group consisting of enzyme treatment, membrane separation, and gel filtration.
(i) Use of a colorant derived from *Ipomoea Batatas* as an edible coloring agent, wherein a concentration of aroma components contained therein is 150 ppm or less when the color value E(10%/1 cm) is 160.
(j) The use according to Item (i), wherein the concentration of aroma components is a total concentration of the following components that can be contained in the colorant: 2-methylbutanol, isoamyl alcohol, tridecane, ethyllactate, hexanol, trans-linalool oxide, benzyl alcohol, phenethyl alcohol, 5-(methylthio)-pentanenitrile, phenylpropanenitrile, ethyl-2-hydroxy-3-phenylpropane, dimethyl phthalate, dihydroactinidiolide, 4-vinylphenol, triethyl citrate, dibutyl phthalate, 4-vinyl-2,6-dimethoxyphenol, phenylacetic acid, ethyl vanillate, 4-ethylcatechol, limonene, cis-pinane, α-terpineol, N-nitrosodibutylamine, guaiacol, 3,7-dimethyloct-1-ene-3,7-diol, 3-hydroxy-α-pyrone, phenol, γ-nonalactone, 4-vinylguaiacol, vanillin, dibutyl phthalate, and acetic acid.

### Examples

The present invention is described in detail below using examples and comparative examples; however, the invention is not limited in any way by these examples.

### Comparative Example 1: Colorant Formulation Derived From Ipomoea Batatas

Ten kilograms of ground tuberous roots of *Ipomoea Batatas* were placed in 20 L of acidic water that had been adjusted to a pH of 2 with sulfuric acid, and allowed to stand overnight at room temperature to extract the colorant. A filter aid and diatomaceous earth were added to the obtained colorant extract, and suction filtration was performed, yielding about 25 L of a plant colorant-containing extract as the filtrate. The colorant component was adsorbed from this extract with Amberlite XAD-7 (a trademark of Organo), which is a synthetic adsorption resin (amount of resin: 3 L, SV = 1), and this resin was thoroughly washed with 10 L of water, after which the colorant was eluted out with a 60 vol% ethanol aqueous solution. The resulting eluate was obtained as an adsorption-treated product of the *Ipomoea Batatas* colorant extract (a primary purified colorant extract: 10 L).

This adsorption-treated product was subsequently concentrated under reduced pressure to obtain 160 g of colorant extract whose color value E(10%/1 cm) was 300. Eighty grams of water and 60 g of ethanol were added to the 160 g of concentrated extract to prepare 300 g of a colorant formulation in solution form whose color value E(10%/1 cm) was 160. When sniffed, this colorant formulation had a vegetable odor characteristic of the plant of the genus C. *Ipomoea.*

### Example 1: Colorant Formulation Derived From Ipomoea Batatas (Solution)

Eight liters of a primary purified colorant extract (adsorption-treated product of *Ipomoea Batatas* colorant extract) obtained by the same method as in the comparative example were treated at 20°C and 3.5 kg/cm² using an ultrafiltration membrane (AHP-2013 Membrane, trademark of Asahi Chemical; molecular weight cut-off: 50,000) (membrane separation treatment). The obtained membrane-treated product that passed through the membrane was subsequently adjusted to a pH of 2.0 with sulfuric acid, and the resulting product was stirred for 30 minutes at a temperature of 40 to 80°C (acid treatment). Five liters of water were subsequently added to this acid-treated product to perform reverse osmosis membrane treatment (NTR-7250 Membrane, trademark of Nitto Denko, molecular weight cut-off: about 3,000), giving 1 L of membrane-treated product that did not pass through the membrane (membrane separation treatment). During this treatment, the aroma components and impurities in the *Ipomoea Batatas* colorant were filtered and removed as the filtrate, and the purified and deodorized colorant component was concentrated as the residue that did not pass through the membrane. This residue was concentrated under reduced pressure to obtain 120 g of concentrate with a color value E(10%/1 cm) of 300 that had been significantly deodorized and purified.

Sixty grams of water and 45 g of ethanol were added to the 120 g of concentrate to prepare 225 g of a colorant formulation (solution) containing the colorant derived from *Ipomoea Batatas,* whose color value E(10%/1 cm) was 160. When sniffed, this colorant formulation was completely odor-free.

### Example 2: Colorant Formulation Derived From Ipomoea Batatas (Solid)

Sixty grams of water and 15 g of dextrin were added to 30 g of a colorant extract concentrate with a color value E(10%/1 cm) of 300, which was prepared by the same method as in Example 1, and had been significantly deodorized and purified. This product was spray-dried to prepare 25 g of a colorant formulation (powder) containing the colorant derived from *Ipomoea Batatas,* whose color value E(10%/1 cm) was 350. When sniffed, this colorant formulation was completely odor-free.

### Example 3: Colorant Formulation Derived From Ipomoea Batatas (Solution)

Eight liters of a primary purified colorant extract (adsorption-treated product of *Ipomoea Batatas* colorant extract) obtained by the same method as in Comparative Example 1 were concentrated under reduced pressure to remove ethanol, and diluted with water to a color value E(10%/1 cm) of 10.

The obtained colorant extract was adjusted to a pH of 3 with sodium hydroxide, after which an acid protease (Newlase F3G (*Rhizopus niveus*)*,* 14,000 u/g; Amano Enzyme) was added in an amount of 0.02 wt%, and the mixture was reacted for 8 hours at 45°C (enzyme treatment). After the reaction, 5 L of water was added to the enzyme-treated product to perform reverse osmosis membrane treatment (NTR-7250 Membrane, trademark of Nitto Denko, molecular weight cut-off: about 3,000), filtering out the aroma components and impurities from the enzyme-treated product, thus giving 1 L of membrane-treated product that did not pass through the membrane (membrane separation treatment). This membrane-treated product was concentrated under reduced pressure to obtain 120 g of concentrate with a color value E(10%/1 cm) of 300 that had been significantly deodorized and purified.

Sixty grams of water and 45 g of ethanol were added to the 120 g of concentrate to prepare 225 g of a colorant formulation (solution) containing a colorant derived from the plant of the genus *C. Ipomoea* whose color value E(10%/1 cm) was 160. When sniffed, this colorant formulation was completely odor-free.

### Example 4: GC-MS Measurement

The amounts of aroma components contained in the colorant formulation produced in Comparative Example 1 (comparative product) and the colorant formulation produced in Example 1 (inventive product) were compared with a gas chromatography-mass spectrometer (GC-MS).

Specifically, 2.5 g of each colorant (color value E(10%/1 cm) = 160) was extracted with 200 mL of diethyl ether containing 0.5 ppm of an internal standard substance (IS: 3-heptanol). This diethyl ether solution was then concentrated under reduced pressure, and the resulting concentrate was placed in a gas chromatography-mass spectrometer (GC-MS) under the following conditions to measure the amount of the aroma components.

### GC-MS measurement conditions:

GC: Agilent 6890
MSD: Agilent 5973N
Column: DB-WAX made by J&W (0.25 mm x 60 m)
Temperature: inlet 250°C, interface 230°C, column temperature 50°C (2 min.) to 220°C, elevation rate 3°C/min.
Split ratio: 70:1
Ionization voltage: 70 eV

The results for the inventive product (Example 1) are shown in Fig. 1, and the results for the comparative product (Comparative Example 1) are shown in Fig. 2. As shown in Fig. 1, the total ion chromatogram of the colorant formulation of Example 1 (inventive product) reveals that the components other than the internal standard substance (IS) were present only in trace amounts (100 ppm or less). In contrast, as shown in Fig. 2, many volatile components were found to be present (total amount: about 600 ppm) in the colorant formulation of Comparative Example 1 (comparative product).

These results were in agreement with the above-mentioned fact that the colorant formulation of Comparative Example 1 had a strong odor (of vegetables or pickles) that is characteristic of *Ipomoea Batatas,* whereas the colorant formulation of Example 1 was odorless.

### Example 5: Flavor Evaluation and Storage Test

The colorant formulation of Comparative Example 1 (comparative product) and the colorant formulation of Example 1 (inventive product) were evaluated for their flavor immediately after manufacture, and their flavor after storage for 15 and 30 days at 5°C, 25°C, or 38°C, by a panel of ten well-trained flavorists.

Moreover, beverages (color value E(10%/1 cm) = 0.04, Brix. 10°, 0.2% citric acid aqueous solution, no flavoring added) was prepared using each of the colorant formulations (comparative and inventive products), and these beverages were comparatively evaluated for their flavor immediately after preparation and after storage for 15 and 30 days at 5°C, 25°C, or 38°C in the same manner as above. The results are shown in Table 1.

**Table 1**

| | | | Immediately After Preparation | After Storage for 15 Days | After Storage for 30 Days |
|---|---|---|---|---|---|
| Example 1 | Formulation | 5°C | A | A | A |
| | | 25°C | A | A | A |
| | | 38°C | A | A | A |
| | Beverage | 5°C | A | A | A |
| | | 25°C | A | A | A |
| | | 38°C | A | A | A |
| Comparative Example 1 | Formulation | 5°C | C | C | C |
| | | 25°C | C | D | E |
| | | 38°C | C | E | E |
| | Beverage | 5°C | B | B | C |
| | | 25°C | B | C | C |
| | | 38°C | B | C | D |

### Evaluation criteria:

A: no odor perceived
B: has a faint off-odor
C: has off-odor
D: has a strong off-odor
E: has a very strong off-odor

As can be seen from the results recorded immediately after preparation in Table 1, the odor of the inventive product was significantly lower than that of the comparative product, and the inventive product was determined to be a substantially odorless colorant. Furthermore, the flavor of the comparative product grew steadily stronger as the storage period lengthened from 15 to 30 days, whereas the inventive product underwent no changes over time, and remained in the same odorless state as immediately after its preparation.

This suggests not only that the *Ipomoea Batatas* colorant formulation of the invention produced by the method of Example 1 contains little or no aroma components that give off an odor, but also that it contains no impurities that would be precursors of these aroma components.

### Example 6: Acerola Drink

### (1) Preparation

An acerola drink was prepared according to the following formulation, using the colorant formulation obtained in Example 3.

| Acerola Drink Formulation: | |
|---|---|
| High Fructose Corn Syrup | 30.0 (wt%) |
| Sugar | 10.0 |
| Citric Acid | 0.4 |
| 1/5 Acerola Juice | 4.4 |
| Acerola Flavor | 0.2 |
| Colorant Formulation of Example 3 | 0.1 |
| Water | 54.9 |
| Total | 100.0 (wt%). |

For comparison, an acerola drink with a substantially similar perceived color density was prepared using the colorant formulation of Comparative Example 1 instead of the colorant formulation of Example 3. The prepared drinks were evaluated for their "cloudiness and presence or absence of a sediment" and "flavor release" immediately after preparation, and after storage for 15 and 30 days at 5°C, 25°C, or 38°C. Similarly, acerola drinks with a substantially similar perceived density were prepared using, instead of the colorant formulation containing the *Ipomoea Batatas* colorant (Example 3), a red-cabbage colorant formulation (tradename: San Red (trademark) RC), a grape juice colorant formulation (tradename: San Red (trademark) GR), and a purple corn colorant formulation (tradename: San Red (trademark) No.5; all of the above are the products of San-Ei Gen F.F.I.). The prepared drinks were evaluated for their "cloudiness and presence or absence of a sediment" and "flavor release" immediately after preparation, and after storage for 15 and 30 days at 25°C. Evaluation was conducted by a panel of ten well-trained flavorists. The results are shown in Table 2.

**Table 2**

| Colorant Used | | Cloudiness/Presence or Absence of Sediment | | | Flavor Release | | |
|---|---|---|---|---|---|---|---|
| | | Immediately after Preparation | After Storage for 15 Days | After Storage for 30 Days | Immediately after Preparation | After Storage for 15 Days | After Storage for 30 Days |
| Example 3 (*Ipomoea Batatas* Formulation) | 5°C | - | - | - | +++ | +++ | +++ |
| | 5°C | - | - | - | +++ | +++ | +++ |
| | 38°C | - | - | - | +++ | +++ | +++ |
| Comparative Example 1 (*Ipomoea Batatas* Formulation) | 5°C | + | ++ | +++ | + | + | ± |
| | 25°C | + | +++ | +++ | + | ± | ± |
| | 38°C | + | +++ | +++ | + | ± | - |
| Red Cabbage Colorant Formulation | 25°C | ± | + | + | ± | - | - |
| Grape Juice Colorant Formulation | 25°C | ++ | +++ | +++ | + | ± | ± |
| Purple Corn Colorant Formulation | 25°C | + | ++ | ++ | + | + | + |

### Evaluation Criteria:

Cloudiness and sediment are indicated by +++>++>+>±>-in decreasing order of amount.
Flavor release was determined based on the five levels of +++>++>+>±>- in decreasing order of flavor release.

As can be seen from the results recorded immediately after preparation that are shown in Table 2, the flavor release of the acerola drink prepared using the colorant formulation of Comparative Example 1 was adversely affected by the vegetable odor originating from *Ipomoea Batatas,* whereas the acerola drink prepared using the colorant formulation (the inventive product) of Example 3 exhibited a significantly improved flavor release compared with that of the acerola drink prepared using the colorant formulation of Comparative Example 1. Moreover, the acerola drink using the colorant formulation of Comparative Example 1 showed further deterioration in flavor release as the storage period lengthened from 15 to 30 days, and underwent significant clouding and sediment formation, while the acerola drink prepared using the colorant formulation of the invention was able to maintain a satisfactory flavor release even after storage at 38°C for 30 days, and exhibited significantly reduced clouding and sediment formation. In contrast, the flavor release of the acerola drink substituting the red cabbage colorant formulation for the colorant formulation of the invention was adversely affected by the odor originating from the red cabbage, and clouding and sediment formation were not sufficiently prevented over time. The acerola drinks using the grape juice colorant formulation and the purple corn colorant formulation were also unable to prevent clouding and sediment formation; further, the acerola drink prepared using the grape juice colorant formulation turned dark red in hue.

### Example 7: Peach Soda for Chu-hai

A peach soda for *chu-hai* was prepared according to the following formulation.

| Peach Soda Formulation: | |
|---|---|
| Colorant Formulation of Example 3 | 0.03 g |
| *Shochu* (Japanese Distilled Spirit) (35% alcohol) | 40.0 ml |
| High Fructose Corn Syrup | 20.0 ml |
| Peach Flavor | 0.3 ml |
| Carbonate | Remainder |
| Total Volume | 100.0 ml. |

The prepared peach soda (alcoholic beverage) was brightly colored purplish red, was free of odor characteristic of *Ipomoea Batatas,* and was excellent in the release of a peach flavor. Further, the prepared alcoholic beverage neither became cloudy nor formed a sediment after storage, and thus had excellent storage stability.

### Example 8: Strawberry Candies

### (1) Preparation

Strawberry candy was prepared according to the following formulation using the colorant formulation obtained in Example 1. Specifically, a mixture of water, sugar, and starch syrup was dissolved by heating to 150°C, and boiled to 100 g. After cooling to 120°C, the colorant formulation (Example 1) was added to the resulting solution together with citric acid and a strawberry flavor. The mixture was molded and then cooled to room temperature to prepare hard candy.

| Strawberry Candy Formulation: | |
|---|---|
| Colorant formulation of Example 1 | 0.05 (g) |
| Sugar | 60.0 |
| Starch Syrup | 40.0 |
| Water | 20.0 |
| Citric Acid | 0.5 |
| Strawberry Flavor | 0.15 |

For comparison, candy with a substantially similar perceived density were prepared using, instead of the colorant formulation of Example 1, the colorant formulation of Comparative Example 1, the red cabbage colorant formulation (San Red (trademark) RC), the monascus colorant preparation (San Red (trademark) MA), grape juice colorant formulation (San Red (trademark) GR), and purple corn colorant formulation (San Red (trademark) No.5).

### (2) Evaluation

Each type of the above strawberry candy was evaluated for their hue, transparency, and flavor according to the following methods.
(a) Hue: The hue of each type of strawberry candy was observed with the naked eye.
(b) Transparency: The transparency of each type of strawberry candy was determined based on the five levels of +++>++>+>±>- in decreasing order of transparency.
(c) Flavor release: Each type of strawberry candy was eaten to make a sensory evaluation of the presence or absence of the flavor release. Flavor release was determined based on the five levels of +++>++>+>±>- in decreasing order of flavor release. The results are shown in Table 3.

**Table 3**

| Colorant Used | Hue | Transparency | Flavor Release |
|---|---|---|---|
| Example 1 (*Ipomoea Batatas* Colorant Formulation) | Bright purplish red | +++ | +++ |
| Comparative Example 1 *(Ipomoea Batatas* Colorant Formulation) | Bright purplish red | +++ | ± |
| Red Cabbage Colorant Formulation | Bright reddish purple | ++ | - |
| Monascus Colorant Formulation | Red | ± | ± |
| Grape Juice Colorant Formulation | Dark red | + | ++ |
| Purple Corn Colorant Formulation | Yellowish red | ++ | + |

As can be seen from Table 3, the flavor release of the strawberry candy prepared using the colorant formulation of Comparative Example 1 was adversely affected by the vegetable odor originating from the *Ipomoea Batatas*, whereas the candy using the colorant formulation of Example 1 (inventive product) exhibited a significantly improved flavor release compared with the candy using the colorant formulation of Comparative Example 1. Further, although it was possible to impart a reddish purple color to candy with the red cabbage colorant formulation, this candy had a poor flavor release due to the odor that is characteristic of the colorant. With the monascus colorant formulation, the color faded upon exposure to light, making it difficult to impart a desired color to candy. With the grape juice colorant formulation, the transparency of the candy was not sufficiently maintained, and the hue of the candy itself turned dark red. With the purple corn colorant formulation, the flavor characteristic of the colorant adversely affected the candy, and a satisfactory flavor release was not obtained.

### INDUSTRIAL APPLICABILITY

The colorant of the invention derived from *Ipomoea Batatas* is a highly purified, and hence an odorless colorant that is free of an unpleasant or off-odor originating from aroma components contained in *Ipomoea Batatas* used as the starting material, or a low-odor colorant in which the odor has been significantly reduced. Further, the *Ipomoea Batatas* of the invention undergoes significantly reduced changes over time, such as odor regression, after heating or long-term storage. Accordingly, when the colorant of the invention or the colorant formulation containing the colorant is used to color beverages and other foods, drugs, quasi-drugs, cosmetics, etc., products with a good flavor can be produced without being affected by the aroma components characteristic of *Ipomoea Batatas.*

## Claims

1. A plant colorant derived from *Ipomoea Batatas,* wherein a concentration of aroma components contained therein is 150 ppm or less when color value E(10%/1 cm) is 160.

2. The plant colorant according to claim 1, wherein the concentration of aroma components is a total concentration of the following components that can be contained in the colorant: 2-methylbutanol, isoamyl alcohol, tridecane, ethyl lactate, hexanol, trans-linalool oxide, benzyl alcohol, phenethyl alcohol, 5-(methylthio)-pentanenitrile, phenylpropanenitrile, ethyl-2-hydroxy-3-phenylpropane, dimethyl phthalate, dihydroactinidiolide, 4-vinylphenol, triethyl citrate, dibutyl phthalate, 4-vinyl-2,6-dimethoxyphenol, phenylacetic acid, ethyl vanillate, 4-ethylcatechol, limonene, cis-pinane, α-terpineol, N-nitrosodibutylamine, guaiacol, 3,7-dimethyloct-1-ene-3,7-diol, 3-hydroxy-α-pyrone, phenol, γ-nonalactone, 4-vinylguaiacol, vanillin, dibutyl phthalate, and acetic acid.

3. A colorant formulation containing the colorant derived from *Ipomoea Batatas* of claim 1 or 2.

4. The colorant formulation according to claim 3, which is in the form of a solution.

5. A method for producing an odorless or low-odor plant colorant derived from *Ipomoea Batatas,* comprising subjecting an adsorption-treated product of an *Ipomoea Batatas* colorant extract to at least one treatment selected from the group consisting of adsorption, ion exchange, acid treatment, extraction, enzyme treatment, and membrane separation.

6. The method according to claim 5, wherein the acid treatment comprises exposing to a pH of 1 to 4 the adsorption-treated product of the *Ipomoea Batatas* colorant extract or a treated product obtained by subjecting the adsorption-treated product to at least one treatment selected from the group consisting of ion exchange, acid treatment, extraction, enzyme treatment, and membrane separation.

7. The method according to claim 5, wherein the enzyme treatment is an acid protease treatment under acidic conditions.

8. A method for producing an odorless or low-odor plant colorant derived from *Ipomoea Batatas*, comprising removing a high molecular weight compound from an adsorption-treated product of an *Ipomoea Batatas* colorant extract, followed by removal of a low molecular weight compound from the colorant extract by a membrane separation treatment.

9. The method according to claim 8, wherein the membrane separation treatment is reverse osmosis using a membrane having a molecular weight cut-off of 2,000 to 4,000.

10. The method according to claim 8, wherein the removal of a high molecular weight compound is at least one treatment selected from the group consisting of enzyme treatment, membrane separation, and gel filtration.

11. The method according to claim 10, wherein the membrane separation treatment used to remove a high molecular weight compound is ultrafiltration using a membrane having a molecular weight cut-off of 10⁴ to 10⁶.

12. The method according to claim 8, wherein an acid treatment is performed prior to, subsequent to, or at the same time as the removal of a high molecular weight compound.

13. A food or a beverage colored with the plant colorant of claim 1 or 2.

14. A method for coloring a food or a beverage comprising adding the plant colorant of claim 1 or 2.

15. The method according to claim 14, wherein the food or beverage is a beverage or a candy.
